# EUROPEAN PATENT APPLICATION

(11) **EP 1 136 567 A1**
(43) Date of publication of application: **26.09.2001**
(21) Application number: 00870057.7
(22) Date of filing: 24.03.2000
(51) Int. Cl.: C12Q 1/68, G01N 33/68

(54) **Method and kit for the screening, the detection and /or the quantification of transcriptional factors**

(71) Applicant: Advanced Array Technologies S.A., 5020 Malonne (BE)
(72) Inventor: Remacle, José, 5020 Malone (BE); Renard, Patricia, 5030 Lonzee (BE); Art, Muriel, 5000 Namur (BE)
(74) Representative: Van Malderen, Joelle

(57) **Abstract**

The present invention is related to a screening, detection and/or quantification method of one or more transcriptional factor(s) (1) possibly present in a biological sample, said method comprising the steps of:
- possibly extracting and isolating said transcriptional factor (1) from said biological sample,
- putting into contact the transcriptional factor (1) with a double-stranded DNA sequence (2) bound to an insoluble solid support (3), and
- detecting and/or quantifying said fixed transcriptional factor (1),
said double-stranded DNA sequence having a specific sequence able to be fixed by the transcriptional factor (1) and being preferably located at a distance of at least about 6,8 nm from the surface of the solid support (3), and said double-stranded DNA sequence being bound to the surface of the insoluble solid support (3) at a concentration of at least 0.01 pmole/cm² of solid support surface (3) .

The present invention is also related to the kit comprising means and media for performing said method.

## Description

### Field of the invention

The present invention is related to a method and kit comprising means and media for the screening, the detection and/or the quantification of transcriptional factors or molecules binding said factors by non radioactive detection means.

### Background of the invention

Transcriptional factors are proteins that bind to specific sequences of DNA, called consensus sequences, and influence the transcription of the DNA into mRNA. Some of these factors directly participate in the transcription process by activating or inhibiting the transcription and regulate the synthesis of proteins needed by cells to function, to adapt, to respond or to differentiate. Some of these proteins have to be transcribed in a constitutive manner (essential role in cell functions) while others are only synthesised in response to specific stimuli or when the cells are for instance in pathological environment. External signals are sensed by receptors and transduced through the plasma membrane followed by cascades of enzymatic kinase reactions, resulting in a phosphorylation or dephosphorylation of the transcriptional factors which affects positively or negatively their binding to their consensus sequence.

An example of drug acting indirectly on transcriptional factor is compactin, an inhibitor of HMG CoA reductase, which leads to an up-regulation of the transcription of the LDL receptor gene, permitting the clearance of cholesterol (US-A-5 563 036).

### State of the art

There is a need for the detection and quantification of transcriptional factors (working as regulators and adaptators) for improving the diagnostic of pathologies or for developing drug affecting their activity.

Several methods are currently used to estimate the activation of transcriptional factors like NFKB. The most common method is to assay for their DNA-binding capacity by gel retardation, also called electrophoretic mobility shift assay (EMSA) (Schreck, R. et al, *Nucleic Acids Res*, **18**(22), 6497-502 (1990)). This method is sensitive, but does not allow the simultaneously processing of numerous samples and requires particular precautions and equipments necessary for the handling of radioactivity.

A second largely used method is based on reporter genes (luciferase or β-galactosidase genes) placed under the control of a promoter containing the consensus sequence. This promoter can be artificial, made of several specific cis-elements and a TATA box, or natural, like the HIV long terminal repeat (LTR) element. However, other transcription factors influence the expression level of the reporter gene. In addition, as the read-out is the enzymatic activity of luciferase, for instance, the results may be affected by interferences with downstream processes like general transcription or traduction machinery. This method is widely used provided the cells are efficiently transfected with the reporter plasmid.

Two other indirect methods are restricted to a few transcription factors. The first one uses antibodies raised specifically against the nuclear localising sequence (NLS) of transcriptional factors like NFKB, a part of the protein which is masked by IκB when the transcription factor is inactivated (Zabel, U. et al., *EMBO J.,* **12**(1), 201-211 (1993)). The activation of NFKB can be visualised by immunofluorescence with this antibody. This method does not suit many samples analysis.

These various methods have been very helpful for fundamental research during these last 10 years, to unravel the molecular activation mechanisms of transcriptional factors like NFκB or AP1. Nevertheless, research has been hampered in this field by the fact that no convenient assay suitable for large scale screening procedure was available.

An assay for testing the inhibition by pharmacological agents of transcriptional factors binding to their specific sequence has been described in the patent US-A-5 563 036. The potentially active agent is incubated with the radioactively labelled transcriptional factor and the inhibition of its binding to the specific sequence is then assayed. This sequence is conjugated to biotin capable of specific binding to avidin immobilised onto microwells. A factor bound to the sequence and bearing biotin will be captured by avidin-coated plates and measured. The labelled protein, the nucleic acid conjugate and the compound form a mixture that is incubated with the avidin immobilised on the solid substrate. The binding of the factor to its sequence is performed in solution for testing possible inhibition by a pharmaceutical agent present in solution.

In solution, the length of the nucleic acid probe ranges between 28 and 60 base pairs. This assay works with high concentration of factor in solution, but it is not at all sensitive for diagnostic assay in biological samples. Furthermore, probe excess has to be added in solution. The probe, which binds to avidin, is preferably a free probe which has a smaller size and thus a much higher diffusion rate, so that the level of binding of probe/factor complex is low.

The patent US-5 747 253 describes a method for the identification of oligomers having a very high specific binding capacity for transcriptional factors. Synthesised oligonucleotides are first incubated with factors in solution in order to test their binding affinity to the factor, then assayed by attaching the oligonucleotide up to 25 bases to a support through a linker moiety present on the oligonucleotides. The reaction between the factor and the oligonucleotide is performed in solution, thereby limiting the sensitivity.

Magnetic microparticles can also be used to capture complexes formed between nucleic acids and proteins as proposed in document US-A-5 939 261. The reaction is performed in solution thus leading to the already mentioned limitations.

Gubler and Abarzua (Biotechniques, 18, 1008, 1011-4 (1995)) describe immobilised antibodies on the surface of wells to capture the transcriptional factor present in a solution. A biotinylated probe is added in the solution so that the active factors will be immobilised together with their probes that can then be detected with alkaline-phosphatase-streptavidin conjugate. However, all transcriptional factors either active or not will bind to the antibodies. There will be a competition for the binding to the antibodies. Results on cell extracts showed a sensitivity of 2 µg in the assay for the p53 protein.

Benotmane *et al* (Analytical Biochemistry 250, 185-185 (1997)) describe a protein-DNA binding of a recombinant protein at equilibrium, said recombinant protein, a fragment 6D3 of HLTF, having a DNA binding domain, the 6D3 portion of an helicase, linked to the glutathione S-transferase (GST). The binding coefficient and the dissociation coefficient in two experimental systems are determined by testing the binding of the biotinylated probe on a protein onto wells surface, or binding of the protein onto a consensus sequence of 27 base pairs fixed to the wells surface. In both cases they detect the protein-DNA binding. However they found a lower dissociation constant in the second test. There was no attempt to test the method on cell extract and it is difficult to assess the real sensitivity of the method given the fact that pure recombinant protein was used in this assay.

### Aims of the invention

The present invention aims to provide a new and improved method and device for the screening, the detection (and possibly the quantification) of transcriptional factors or molecules binding said factors, preferably several of them present in a biological sample, preferably by non radioactive means.

### Summary of the invention

The present invention is related to a screening, detection and/or quantification method of one or more transcriptional factor(s), possibly present in a biological sample, said method comprising the step of putting into contact the transcriptional factor with a double-stranded DNA sequence bound to an insoluble solid support, said double-stranded DNA sequence having a specific nucleotide sequence able to be fixed (recognised specifically through known biochemical interactions) by said transcriptional factor, said specific sequence being advantageously located at a distance of at least 6,8 nm, preferably higher than 8 nm, from the surface of the solid support; said double-stranded DNA being present upon the surface of the solid support at the concentration of at least 0.01 pmole/cm², preferably at least 0,1 or more pmole/cm² of the solid support surface.

The method according to the invention comprises also the step of screening or detecting the fixed transcriptional factor and possibly quantifying the presence of said fixed transcriptional factor (and possibly quantifying the concentration of the trancriptional factor present in the biological sample).

The present invention is based on the specific interaction between a transcriptional factor 1 and its consensus DNA sequence 2 previously immobilised on a surface of an insoluble solid support 3 (step 1), said immobilisation of the consensus DNA sequence 2 upon a solid support being obtained previously by the interaction between a double-binding pair 6, 7 (biotin/streptavidin, antigen/antibody, hapten/receptor,...) the consensus DNA sequence 2 being labelled with the first binding element (biotin) 6 and the surface of the insoluble solid support 3 being bound with the second binding element (streptavidin) 7.

After washing, the presence of the fixed transcriptional factor 1 is detected preferably by using firstly a primary antibody 4 raised against the factor 1 (step 2) and then a secondary labelled antibody 5 (in this example conjugated with peroxidase (step 3)) directed against the primary antibody 4.

The presence of the peroxidase is then advantageously assayed by a colorimetric detection of the reaction product in solution (step 4). Such non-radioactive method is rapid, more sensitive than gel retardation, and suits for large-scale screening or detection upon various solid supports. The method can be also adapted for the screening and the recovering of molecules (or compounds) binding to said transcriptional factor(s), preferably by using specific steps, means and media, such as the ones described in the US patent 5,563,036. Said molecules or compounds binding to the transcriptional factor(s) are advantageously a molecule or compound which inhibits the binding of the transcriptional factor 1 to its specific nucleotide sequence and said method comprises the step of forming a mixture by combining a labelled protein comprising a portion of the transcriptional factor, the specific nucleotide sequence being bound to the insoluble solid support according to the characteristics of the present invention. Thereafter, the method comprises the step of incubating said mixture in the conditions whereby in the absence of the molecule or compound, the labelled protein is binding to the specific nucleotide sequence, the step of separating from the solid support a fraction of said mixture, whereby the fraction comprises the labelled protein if said labelled protein is not bound to the specific sequence of the transcriptional factor according to the invention, and the step of detecting the presence or absence of said labelled protein upon said solid support, wherein the absence of said detected label on said solid support means that the compound or molecule inhibits the binding of a transcriptional factor to its specific nucleotide sequence, the labelling being preferably a non radio active labelling. Preferably, said method comprised also the step of recovering the detected molecule or compound.

Said method could be adapted for a specific screening kit or device comprising all the means and media for performing said method, preferably in high-throughput screening device, comprising computer-controllable electromagnetic means and robots allowing the screening and detection upon any type of solid support.

The use of consensus DNA sequence already fixed on the support allows an industrial production with reproducible and quality control of the kit containing such prepared support. It also simplifies the use of the kit since the biological sample can be incubated directly with such prepared surface without requiring the addition of any other reagent.

Said general method for detecting the DNA-binding capacity of transcriptional factors is enough sensitive, specific and valid for most if not all transcription factors. Unexpectedly, it is possible to obtain such sensitive method by increasing the spacer between a consensus sequence on which the transcriptional factor will bind and by using a very high density of these sequences attached on the surface. The method is so sensitive that it can be used for the detection of one factor present in a biological sample, but also for the detection of several factors by the use of multiple probes attached in distinctive spots on a surface such as biochips.

The minimum spacer was found to be a nucleotide sequence, such as a DNA sequence, of 20 base pairs (bp) but said spacer has preferably between 50 and 150 base pairs according to the type of solid support used. Experimentally, probes of 72 or 122 bp gave the highest signal to detect NFKB (example 1). There is however a maximum size for the spacer and experimentally the signals obtained with spacer of 150 and 255 bp gave lower signal in multiwell assay. However, for surfaces like glass used in microarrays, the binding of these two very long probes was found beneficial. The presence of the consensus sequence at long distance from the surface can be obtained similarly through the presence of a chemical spacer of at least 10 atoms, preferably of at least 44 atoms, more preferably of at least 50 atoms.

The present invention is also related to a screening, diagnostic and/or quantification kit (or device, including high-throughput screening device) comprising means and media for performing the method according to the invention, preferably at least one double-stranded DNA sequence bound to the surface of an insoluble solid support, said double-stranded DNA sequence having a specific nucleotide sequence able to be fixed (recognised) by a transcriptional factor, said specific sequence being located at a distance of at least 6,8 nm (at least 44 carbon atoms, more preferably at least about 8 nm or 50 carbon atoms) from the surface of the solid support, said double-stranded DNA sequence being bound to the insoluble solid support at a concentration of at least 0.01 pmole, preferably at least 0.1 pmole or more (0.5 to 5 pmoles/ cm²) of solid support surface.

The screening, detection and/or quantification kit (device) according to the invention comprises also all the elements necessary for the specific screening, detection and/or quantification of a fixation (binding) between the transcriptional factor and the double-stranded DNA by a suitable method, especially the ones well known by the person skilled in the art.

Said screening, detection and/or quantification method and kit (device) are suitable for the (possibly simultaneous) detection and/or quantification of multiple different transcriptional factors present in the same biological sample and possibly upon the same solid support and for the screening or detection of molecules or compounds binding said transcriptional factor(s) able to inhibit or activate the binding of said transcriptional factor(s) to its specific nucleotide sequence.

Said screening, detection and/or quantification method and kit (device) are suitable for testing the activity of pharmaceutical drugs or methods acting upon these transcriptional factors (i.e. as inhibitors and/or activators of their binding to the DNA).

The invention is also related to said compound or molecule identified or recovered by said method and possibly integrated in a pharmaceutical composition for preventing or treating various symptoms or diseases.

Said screening, detection and/or quantification method and kit are suitable for detection and quantification of all the transcriptional factors for which the DNA binding sequence is known. These transcriptional factors and their properties such as their consensus binding sequence are listed in the Web site " http://transfac.gbf-braunschweig.de/TRANSFAC/cl.cl.html" The most commonly tested factors are selected from the group consisting of NF-KB, AP-1, CREB, SP-1, C/EBP, GR, HIF-1, Myc, NF-AT, Oct, TBP and CBF-1.

NFκB is an important ubiquitous transcriptional factor activated following cell stimulation, involved in the immune response to some viral and bacterial products, oxidative stresses or pro-inflammatory cytokines (Baeuerle, P. A. and Baichwal, V. R., *Adv Immunol,* **65**, 111-37 (1997)).

AP1 is also dimeric transcriptional factor which is involved in many cell responses through activation of kinase cascade. This transcription factor is involved in a variety of biological processes like cell growth, differentiation, or apoptosis.

Preferably, said detection and possibly quantification is obtained by the use of molecules that are able to specifically bind to the transcriptional factor fixed upon the double-stranded DNA, such as (preferably monoclonal) antibodies or specific hypervariable portions thereof (Fab', Fab2', etc.).

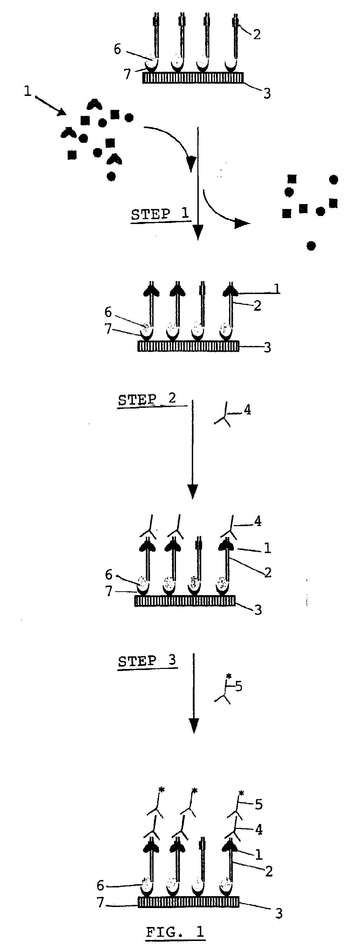

According to a preferred embodiment of the present invention, said binding is followed by incubation (and washing) with labelled molecules able to react with the first molecule binding to said transcriptional factor, preferably (monoclonal) antibodies directed against the anti-transcriptional factor antibodies or specific hypervariable portions thereof (Fab', Fab2', etc.). Said last antibodies are preferably labelled with non radioactive markers allowing a detection, preferably by colorimetry, fluorescence or precipitation of a metal deposit (such as silver staining). Said non radioactive test is preferably based upon a colorimetric assay as described in the enclosed Fig. 1.

The selected antibody recognises an epitope which is accessible when the factor is in its active form and bound to DNA. Usually, the active and inactive forms of the factors differ in their phosphorylation state or by the presence of an inactivator protein. The antibody has to recognise the active form. The concentration of these transcription factors in a cell is very low and may reach the limit of the affinity coefficient of the antibodies. An antibody with a high affinity (i.e. with very low coefficient of dissociation) can highly increase the signal obtained. However, any other protein which has an affinity for the factor can be used for the detection.

Special applications are the use of specific antibodies directed against phospho-protein epitopes or against specific proteins which are part or can be attached to the transcriptional factor. In example 4, antibodies are used against CREB or P-CREB. For NF-κB, antibodies directed either against P-50 or P-65 allow to determine the pattern of association of proteins giving the NF-κB activity.

The attached transcriptional factor can also be used to test for the presence of proteins which have affinity for the transcriptional factor.

If the binding is specific and if no other proteins are used on the nucleotide sequence binding on the surface, for example when the nucleotide sequences are directly linked to said surface, then a direct detection of protein will give an estimation and/or quantification of the bound factor.

A signal obtained in each well or for each spot of a biochip is recorded and the means of the signal are calculated for each identical consensus sequence. Usually, two (and preferably three to five) identical spots are present on each array in order to correct variations that may occur at any step of the process. The background values are wells or spots coated with a consensus sequence, in which binding buffer and lysis buffer is added instead of a cell extract or purified transcription factor (negative control). A positive control is preferably added as a factor obtained in a pure active form and for which a consensus nucleotide sequence is attached to the surface of the solid support in the test.

Quantification has to take into account not only for the binding yield but also the detection part of the process and the reading scale. Standards using purified active transcriptional factor are very useful since they can be added to the sample at either step of the process in given amount as reference value to which the results will be compared. These purified proteins can be used as internal or external standards. Validation of the use of these standards has to be performed earlier in order to validate their binding efficiency compared to the tested factors.

In the case of biological applications, comparison of transcriptional factors involved in the cell response can be quantified in comparison to transcriptional factors which are constitutively active (there are factors which regulate the expression of house keeping genes). The use of these "relative" quantification simplifies the assays since all factors are treated in the same way during the various steps.

The solid support (biochip) can be inserted in a support connected to another chamber and automatic machine through the control of liquid solution based upon the use of microfluidic technology. By being inserted into such a microlaboratory system, it can be incubated, heated, washed and labelled by automates, even for previous steps (like an amplification by PCR) or the following step (labelling and detection). All these steps can be performed upon the same solid support.

The binding of the double-stranded DNA sequence to the insoluble solid support is preferably made through an interaction between a coupling pair, such as biotin/streptavidin, antigen/antibody, ligand/receptor,... coupling pairs, the double-stranded DNA being preferably bound to biotin and streptavidin being fixed upon the surface of the solid support in order to allow a specific binding through the coupling pairs.

According to the invention, the solid support is preferably selected from the group consisting of filters, glass, metallic supports, polymeric supports (preferably a polystyrene support activated with carboxyl or amino groups present on its surface) or any other support used in the microchips (or arrays) technology (preferably activated glass bearing aldehyde groups), said support comprising also specific coatings, markers or devices (bar codes, electronic devices,... for improving the assay.

If glass presents many advantages (like being inert and having a low auto-fluorescence), other supports like polymers can be useful since they can also be obtained with various chemically well-defined groups at their surface, thus allowing the binding of the nucleotide sequence.

The inventors found that given the high sensitivity of the method, it is possible to use the microchips technology to assay transcriptional factors. Miniaturisation allows to perform one assay onto a surface (usually circular spots of about 0.1 to about 1 mm diameter). A low density array, containing 20 to 400 spots is easily obtained with pins of 0.25 mm at low cost. Higher density of spots going to 1,600 spots per cm2 can be obtained by reducing the size of the spots for example to 0.15mm. The advantages of this technology is the high number of data which can be obtained and analysed simultaneously, the possibility to perform replicates and the small amount of biological sample necessary for the assay.

The arrays technology allows the simultaneous detection of different factors present in the same sample, which is possible due to the fact that several factors bind to their consensus sequence in the same reacting conditions. Solutions are available for example from Promega (Madison, Wisconsin, USA) for the binding of several factors (Catalog E3581). Conditions which allow the binding of the factors studied may be optimised by the person skilled in the art. Other binding assays involving DNA-DNA recognition, antigen-antibody or receptor-ligand recognition can be performed simultaneously on the same chips for other molecules.

Preferably, the diagnostic and/or quantification kit (device) is a biochips comprising a solid support with an array surface of at least 4, 10, 20, 50, 100, 1000 or 10000 regions/cm², each of said discrete regions being covered with at least one double-stranded DNA sequence having a specific nucleotide sequence able to be fixed by a transcriptional factor and said specific sequence being located preferably at a distance of at least 6,8 nm from the surface of the solid support, said double-stranded DNA sequence being present upon the surface of the solid support, preferably in each region, at the concentration above-described.

Said double-stranded DNA is preferably fixed covalently to the solid support or by any of the above cited methods.

The detection of the specific binding between the transcriptional factor and the double-stranded DNA sequence is preferably obtained by any one of the methods described previously, preferably by a method allowing a precipitation of a metal deposit (such as silver staining) at the location where the transcriptional factor has fixed (recognised) the double-stranded DNA sequence).

As only activated transcriptional factors are captured by the nucleotide sequences bound to the solid support (preferably microwell plates or microarray), the design of these sequences and binding conditions allow a very high sensitivity necessary for assays in biological samples.

The present invention will be described in more details in the following examples in reference to the enclosed drawings.

### Short description of the drawings

Figure 1 is a schematic presentation of the procedure for the assay of a transcriptional factor.

Figure 2 represents a DNA-binding assay of NFκB from cell extract in microwells containing a 122 bp DNA probe incubated with increasing amounts of cell lysates coming either from unstimulated cells (white columns) or from IL-1-stimulated cells (dark columns). The values represent the mean of 3 values ± S.D. EMSA performed on the same samples and the detection limit was obtained at 5 µg per well. The detection limit for the EMSA was 1 µg per well.

Figure 3 represents a detection of NFκB on microarray by using spotted DNA sequences of different lengths containing a consensus sequence for NFκB.

Figure 4 presents the values obtained for the detection of NFκB present in cells stimulated or not with IL-1 on microarray by using DNA probes of different lengths containing a spotted consensus sequence for NFκB.

### Examples

### Example 1: Microwell colorimetric NFKB assay. Effect of the capture length

(The steps are presented in figure 1).

### Step 1 : Binding of the double strand oligonucleotidic probe on multi-well plates

The spacer double strand nucleotide sequences were constructed from the following CMV sequence: 5'TGGCCAAGCGGCCTCTGATAACCAAGCCTGAGGTTATCAGTGTAATGAAGCGCCGCA TTGAGGAGATCTGCATGAAGGTCTTTGCCCAGTACATTCTGGGGGCCGATCCTCTGAGA GTCTGCTCTCCTAGTGTGGATGACCTACGGGCCATCGCCGAGGAGTCAGATGAGGAAGA GGCTATTGTAGCCTACACTTTGGCCACCGCTGGTGTCAGCTCCTCTGATTCTCTGGTGT CACCCCCAGAGTCCCCTGTAC linked to the NFκB consensus oligonucleotide 5'AGTTGAGGGGACTTTCCCAGGC-3'. The probes contained either 0, 50, 100, 150 or 255 bp of the pre-cited CMV sequence 5' extremity, followed by the NFκB consensus sequence, resulting respectively in 22, 72, 122, 172 or 277 bp length. The CMV extremity is 5' biotinylated, so that these probes can be linked to streptavidin-coated 96-wells plates : 2 pmoles of probes per well are incubated lh at 37 °C in 50 µl 10 mM phosphate buffer 150 mM NaCl (hereafter called PBS₁₅₀). Plates are then washed and the amount of DNA fixed on streptavidin-coated plates was quantified using the picogreen assay (Molecular Probes, OR, USA).

### Step 2 : Binding of NFκB on the double strand probe

This assay was first performed with purified p50-p50 (Promega, Madison, WI, USA), and then with whole cell lysates from SV-40 transformed WI-38 fibroblasts (WI-38 VA13 cell line) stimulated with IL-1β or left unstimulated. 20 µl of p50 diluted in lysis buffer or 20 µl of whole cell extract are incubated with 30 µl of binding buffer (AAT, Namur) in microwells coated with the NFκB binding consensus oligonucleotide. After 1h incubation at room temperature with a mild agitation (200 rpm on IKA MS2 vortex, Germany), microwells are washed 3 times for 2 min with 10 mM phosphate buffer 50 mM NaCl (hereafter called PBS₅₀) + Tween 0.1%, and once with PBS₅₀ alone.

### Step 3 : Binding of anti-NFκB on the NFκB-DNA complex

100 µL of diluted rabbit anti-NFκB antibodies (#sc-372, Santa Cruz, CA, USA) diluted 1000 times in PBS₅₀ and 1% non fat dried milk are incubated in each well for lh at room temperature. Microwells are then washed with 200 µl PBS₅₀ + 0.1 % Tween.

### Step 4 : Binding of peroxidase-conjugated anti-rabbit IgG on anti-NFκB p50

100 µL of diluted peroxidase-conjugated anti-rabbit IgG (#611-1302, Rockland, Gilbertsville, PA, USA) diluted 1000 times in PBS and 1% non fat dried milk are incubated in each well for lh at room temperature. Microwells are then washed with 200 µl PBS₅₀ + 0.1 % Tween.

### Step 5 : Colorimetric revelation

100 µl of tetramethylbenzidine (Biosource, Belgium) are incubated 10 min at room temperature before adding 100 µl of stopping solution (Biosource, Belgium). Optical density is then read at 450 or 405 nm, using a 655 nm reference wavelength, with an Ultramark microplate reader (Biorad, CA, USA).

### Example 2: Assay for NFκB in biological samples

### Cell culture and preparation of cell extracts

The cell line WI-38 VA13, a SV40 virus-transformed human fibroblastic cell line, was purchased from the American Type Culture Collection and plated in 75 cm² flasks. Cells were serially cultivated in Minimum Essential Medium (Gibco, UK) supplemented with Na₂SeO₃ 10⁻⁷ M and antibiotics, in the presence of 10 % fetal bovine serum. To activate NFκB, cells were stimulated for 30 min with 5 ng/ml IL-1β (R&D systems, UK). After stimulation, cells were rinsed twice with cold PBS₁₅₀ before being scraped and centrifuged for 10 min at 1000 rpm. The pellet was then resuspended in a 100 µl lysis buffer containing a (Hepes 20 mM pH 7.5, NaCl 0.35 M, glycerol 20 %, NP-40 1 %, MgCl₂.6H₂O 1 mM, EDTA 0.5 mM, EGTA 0.1 mM) containing one detergent and protease and phosphatase inhibitors. After 10 minutes on ice, the lysate was centrifuged for 20 min at 14000 rpm. The supernatant constitutes the total protein extract and can be kept frozen at -70°C.

### Assay for NFκB

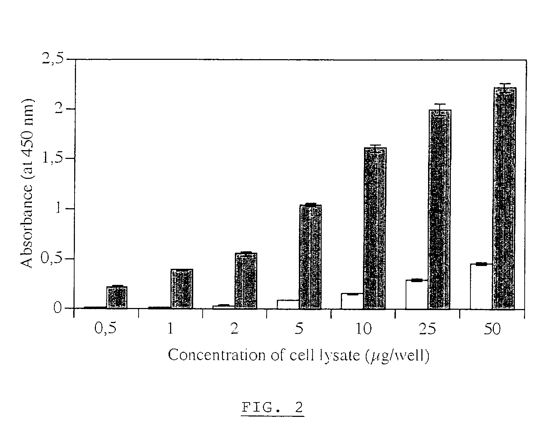

Cell protein extracts are coming either from unstimulated cells or from cells stimulated with IL-1 in order to proceed for the assay as described in the example 1. In this experiment the concentrations ranged between 5 and 50 µg per well. The assay was performed in parallel with EMSA, on the same protein extracts. The results presented on figure 2 clearly show that the NFκB DNA-binding assay in microwells is more than 10 times more sensitive than the EMSA : 5 µg of proteins were required to detect a first detection signal by EMSA, but less than 0.5 µg of proteins was necessary for the microwell assay.

### Example 3 : Microwell colorimetric AP-1 assay

### Step 1 : Binding of the double strand oligonucleotidic probe on multi-well plates

The spacer double strand probes were constructed from the CMV sequence given in example 1 and linked to the AP-1 (21 bp) consensus oligonucleotide 5'-CGCTTGATGAGTCAGCCGGAA-3' (Promega, Madison, WI, USA). The probes contain 100 bp of the pre-cited CMV sequence 5' extremity. The CMV extremity is 5'biotinylated, so that these probes can be linked to streptavidin-coated 96-wells plates (Roche, Germany). The binding of the probe on multiwell plate was performed as for NFκB.

### Step 2 : Binding of AP-1 on the double strand probe

This assay was performed with purified c-Jun/c-Jun (#3061, Promega, Madison, WI, USA), and with nuclear extracts from WI-38 VA 13 fibroblasts stimulated or not with PMA. 10 µl of either the c-Jun/c-Jun solution diluted 10 times or containing 5 µg protein of nuclear extract are first incubated 10 min with 40 µl of binding buffer (AAT, Namur, Belgium) and then added in microwells coated with the AP-1 binding consensus oligonucleotide. After a lh incubation at room temperature with a mild agitation (200 rpm on IKA MS2 vortex, Germany), microwells are washed 3 times with PBS₅₀ + Tween 0.1%, and once with PBS₅₀ alone.

### Steps 3, 4 and 5 : Binding of anti-Jun on the AP-1-DNA complex and detection

The binding of the first antibody specific for c-jun (Santa Cruz, CA, USA SC-1694), of the second antibody conjugated with peroxidase and the detection of the resulting product of the peroxidase activity was performed as described above for NF-κB.

The results showed a clear detection of the AP-1 both on the preferred c-jun/c-jun and on the stimulated fibroblasts.

### Example 4 : Microwell colorimetric CREB and phospho-CREB assay

The spacer double strand probes were constructed from the CMV sequence as for NF-κB of example 1.This sequence was linked to the CREB (27bp) consensus oligonucleotide 5'-AGAGATTGCCTGACGTCAGAGAGCTAG-3' (Promega, Madison, WI, USA). The probes contain 100 bp of the pre-cited CMV sequence 5' extremity. The CMV extremity is 5'biotinylated, so that these probes can be linked to streptavidin-coated 96-wells plates (Roche, Germany). The binding of the probe on multiwells plate was performed as for NFκB (step 1)

The binding of CREB and phospho-CREB on the double strand probe (step 2) was performed with nuclear extracts from L 929 murin fibrosarcoma. 5 µg protein of nuclear extract are incubated 60 min in 50 µl of binding buffer in microwells coated with the CREB binding consensus oligonucleotide. After a 1h incubation at room temperature with a mild agitation (200 rpm on IKA MS2 vortex, Germany), microwells are washed 3 times with PBS + tween 0,1%, and once with PBS alone.

The binding of the first antibody specific for CREB (step 3) (New England Biolabs,Beverly, MA, USA) and phospho-CREB (New England Biolabs,Beverly, MA, USA), of the second antibody conjugated with peroxidase (Step 4) and the detection of the resulting product of the peroxidase activity (step 5) was performed as described above for NF-κB. The results gave a signal of 0.021 OD for the control and 0.226 for the L929 cells. The use of an antibody specific for phospho-CREB gave value of 0.207.

### Example 5 : Microarray NFκB assay: on purified p50 homodimer and on biological samples

### Step 1 : Binding of the probes on glass

Activated glass slides bearing aldehyde groups were purchased from CEL Associates (USA).

The plates were first incubated in 20 ml of a 50 µg/ml streptavidin solution in a 10 mM phosphate buffer (pH 7.4) containing 10 mM NaCl (PBS₁₀ buffer). After lh incubation at 20°C, the plates were washed 3 times for 2 min in PBS₁₅₀ containing 0.02% Tween 20 and then 2 times 2 min with water. The plates were then incubated for 2h at 20°C in 20 ml of PBS₁₅₀ solution containing 10 % non fat dried milk and then washed 5 times 2 min in PBS₁₅₀ solution.

Spotting was performed with the NFκB consensus probe with spacers of 50, 100, 150 and 250 bp as in example 1. The capture probes were printed onto the silylated microscopic slides with a home made robotic device. 250 µm pins from Genetix (UK) were used. The spots have an average of 400 µm in diameter and the volume dispensed is about 1 nl. Various solution concentrations of probes were used ranging from 150 to 3000 nmolaire. Slides were incubated for 1h at room temperature, washed twice for 2 min with PBS₅₀ containing 0.02% Tween 20 and then 3 times for 2 min in water.

### Step 2 :Binding of NFκB on the double strand probe fixed on micro-array

This assay was first performed as above with purified p50 homodimer (Promega, Madison, WI, USA), and then with whole cell lysates from WI-38 VA 13 fibroblasts stimulated with IL-1β or left unstimulated. The control was 20 µl of lysis buffer mixed with 30 µl of binding buffer. 20 µl of p50 diluted in lysis buffer or 20 µl of cell extract are incubated with 30 µl of binding buffer (AAT, Namur, Belgium) per incubation chamber. After lh incubation at room temperature with a mild agitation (200 rpm on IKA MS2 vortex, Germany), the arrays were washed 3 times.

### Steps 3, 4 and 5 : Binding of anti-NFκB on the NFκB-DNA complex and detection

The binding of the Primary anti-NFκB antibody was then used for reaction on the factor followed by a washing and an incubation with a secondary antibody. In this case, the second antibody was gold-labelled. After 3 incubations of 10 min in a mixture of silver enhancer A, the slides were washed with water.

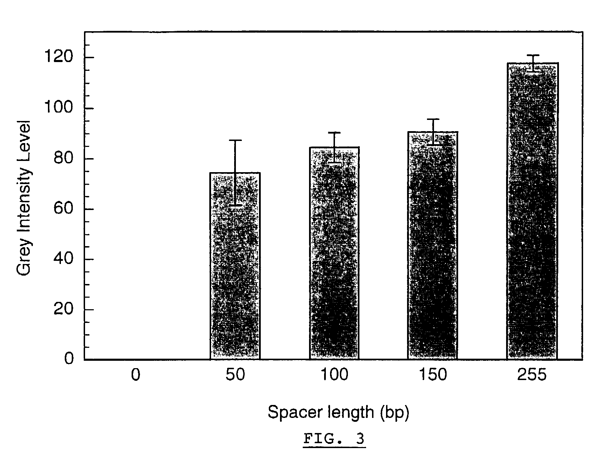

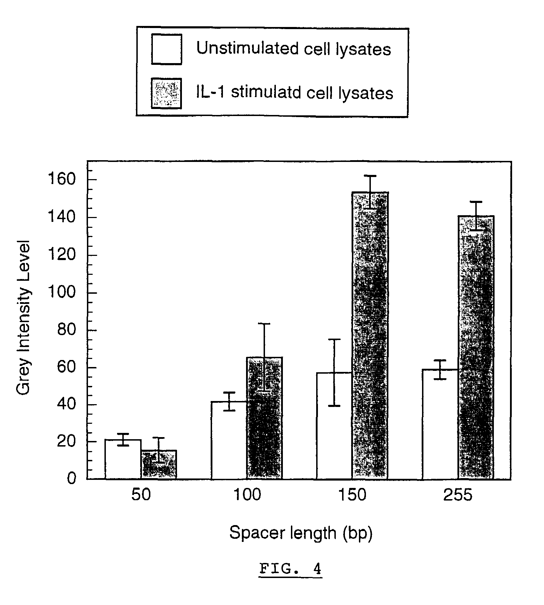

There was no signal for the control (A) and the consensus probe without spacer (B). The signal increases with the spacer length and the concentration. It became easily detectable with the 50 bp spacer as show in figure 3 presented at spotting concentration of 0.6 υM equivalent per cm². Activated NF-κB is also detectable in cell lysates stimulated with IL-1 (B), with a much higher intensity than in unstimulated cells (A) (figure 4). Similarly to the p50 homodimer, the signal of intracellularly activated NFκB increases with the probe length.

## Claims

1. A screening, detection and/or quantification method of one or more transcriptional factor(s) (1) possibly present in a biological sample, said method comprising the steps of:
- possibly extracting and isolating said transcriptional factor (1) from said biological sample,
- putting into contact the transcriptional factor (1) with a double-stranded DNA sequence (2) bound to an insoluble solid support (3), and
- detecting and/or quantifying said fixed transcriptional factor (1),
characterised in that said double-stranded DNA sequence is bound to the surface of the insoluble solid support (3) at a concentration of at least 0.01 pmole/cm² of solid support surface (3).

2. The method according to claim 1, characterised in that the specific sequence of the double-stranded DNA sequence (2)is located at a distance of at least 6,8 nm from the surface of the solid support.

3. The method according to claim 1 or 2, characterised in that the detection and/or the quantification of the fixed transcriptional factor (1) is obtained by a specific binding of said fixed transcriptional factor with non radioactive labelled molecules.

4. The method according to any of the preceding claims 1 to 3, for the (possibly simultaneous) detection and/or quantification of multiple different transcriptional factors (1) present in the same biological sample.

5. The method according to any of the preceding claims 1 to 4, wherein the solid support (3) is an array bearing upon at least 4 spots/cm2 of solid support surface containing said double-stranded DNA sequences for the binding of the transcriptional factor(s) (1).

6. The method according to any one of the preceding claims, characterised in that the double-stranded DNA sequence (2) comprises, between the specific sequence to be fixed by the transcriptional factor and the solid support (3), a spacer of at least 13.5 nm.

7. The method according to claim 6, characterised in that said spacer is a double-stranded DNA nucleotide sequence of at least 20 base pairs, preferably at least 40 base pairs.

8. The method according to any one of the preceding claims, characterised in that the double-stranded DNA sequence (2) is bound to a first member of a binding pair, preferably biotin (6), able to interact with a second member of said binding pair, preferably streptavidin (7), bound to insoluble solid support (3).

9. The method according to any one of the preceding claims, characterised in that the double-stranded DNA sequence (2) is covalently bound to the surface of the insoluble solid support (3).

10. The method according to any of the preceding claims, characterised in that it comprises the steps of screening, detecting and/or quantifying molecules or compounds binding to said transcriptional factor or inhibiting the binding of the transcriptional factor (1) to its specific sequence of the double-stranded DNA sequence (2) bound to the solid support (3).

11. A method according to any of the preceding claims characterised in that it comprises the step of identification of the transcriptional factor and/or of the proteins which are part of its active complex.

12. A screening, diagnostic and/or quantification kit comprising means and media for performing the method according to any one of the preceding claims.

13. A screening, diagnostic and/or quantification kit according to claim 12 of a transcriptional factor or a molecule able to bind to said transcriptional factor or inhibit the binding of said transcriptional factor to its specific nucleotide sequence, characterised in that it comprises a double-stranded DNA sequence (2) bound to an insoluble solid support (3), said double-stranded DNA sequence having a specific nucleotide sequence, said specific nucleotide sequence being preferably located at a distance of at least about 6.8 nm from the surface of the solid support (3) and said double-stranded DNA sequence being present upon the surface of the solid support at a concentration of at least 0.01 pmole/cm² of solid support surface (3).

14. Use of the screening, diagnostic and/or quantification kit according to the claim 12 or 13, for the screening, the detection and/or the quantification of a transcriptional factor present in a biological sample, preferably a transcriptional factor selected from the group consisting of NF-KB, AP-1, CREB, SP-1, C/EBP, GR, HIF-1, Myc, NF-AT, Oct, TBP and CBF-1.

15. Use of the screening, diagnostic and/or quantification kit according to the claim 12 or 13, for the screening, the detection and/or the quantification of a molecule able to bind to a transcriptional factor or able to inhibit the binding of a transcriptional factor to its specific nucleotide sequence, said transcriptional factor being preferably selected from the group consisting of NF-KB, AP-1, CREB, SP-1, C/EBP, GR, HIF-1, Myc, NF-AT, Oct, TBP and CBF-1.


### Annex to the application documents - subsequently filed sequences listing

SEQUENCE LISTING

<110> Advanced Array Technologies S.A.

<120> METHOD AND KIT FOR THE SCREENING, THE DETECTION AND/OR
      THE QUANTIFICATION OF TRANSCRIPTIONAL FACTORS

<130> P.AAT.02/EP

<140> 00870057.7
<141> 2000-03-24

<160> 4

<170> PatentIn Ver. 2.1

<210> 1
<211> 255
<212> DNA
<213> Cytomegalovirus

<400> 1
tggccaagcg gcctctgata accaagcctg aggttatcag tgtaatgaag cgccgcattg 60
aggagatctg catgaaggtc tttgcccagt acattctggg ggccgatcct ctgagagtct 120
gctctcctag tgtggatgac ctacgggcca tcgccgagga gtcagatgag gaagaggcta 180
ttgtagccta cactttggcc accgctggtg tcagctcctc tgattctctg gtgtcacccc 240
cagagtcccc tgtac                                                  255

<210> 2
<211> 22
<212> DNA
<213> Artificial Sequence

<220>
<223> Description of Artificial Sequence: NFKappaB
      consensus oligonucleotide

<400> 2
agttgagggg actttcccag gc                                          22

<210> 3
<211> 21
<212> DNA
<213> Artificial Sequence

<220>

<223> Description of Artificial Sequence: AP1 consensus
      oligonucleotide

<400> 3
cgcttgatga gtcagccgga a                                                21

<210> 4
<211> 27
<212> DNA
<213> Artificial Sequence

<220>
<223> Description of Artificial Sequence: CREB consensus
      oligonucleotide

<400> 4
agagattgcc tgacgtcaga gagctag                                          27